(19) Europäisches Patentamt
European Patent Office
Office européen des brevets

(11) **EP 4 691 361 A1**

(12) **EUROPEAN PATENT APPLICATION**

(43) Date of publication:
**11.02.2026 Bulletin 2026/07**

(21) Application number: 24382899.3

(22) Date of filing: **09.08.2024**

(51) International Patent Classification (IPC):
*A61B 5/07* *(2006.01)*     *A61B 5/145* *(2006.01)*

(52) Cooperative Patent Classification (CPC):
**A61B 5/14539; A61B 5/073; A61B 5/14507;**
**A61B 5/14546;** A61B 5/4238

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Fundación Tecnalia Research &**
**Innovation**
**20009 Donastia-San Sebastían Gipuzkoa (ES)**

(72) Inventors:
 • **PEÑA DIAZ, Marina**
 **20009 San Sebastián - Guipúzcoa (ES)**

 • **GARCIA LIZARRIBAR, Andrea Alicia**
 **20009 San Sebastián - Guipúzcoa (ES)**
 • **IBARLUCEA CANTON, Bergoi**
 **20009 San Sebastián - Guipúzcoa (ES)**
 • **HERNANDEZ JIMENEZ, Erik**
 **20009 San Sebastián - Guipúzcoa (ES)**
 • **GARCIA MENDIZABAL, Beñat**
 **20009 San Sebastián - Guipúzcoa (ES)**
 • **BRIZ ICETA, Nerea**
 **20009 San Sebastián - Guipúzcoa (ES)**

(74) Representative: **Balder IP Law, S.L.**
**Paseo de la Castellana 93**
**5ª planta**
**28046 Madrid (ES)**

(54) **A SYSTEM COMPRISING A SWALLOWABLE OR SUCKABLE DEVICE**

(57) The invention relates to a system comprising:
• a swallowable or suckable device for taking measurements in a part of a digestive system, the device comprising:
◦ a sensor configured to measure a biomarker by providing measurements of electrical parameters;
◦ at least one of a data storage and a wireless communication module for receiving measurements taken by the sensor, the wireless communication module being configured to transmit data outside of the device;

◦ an energy source for providing energy to the sensor and to the at least one of a data storage and a communication module; and
◦ a cover, the cover housing the sensor, the energy source and the at least one of a data storage and a communication module

• processing means configured to receive the measurements of the sensor and to process the measurements to estimate a presence of a biomarker.

EP 4 691 361 A1

## Description

### TECHNICAL FIELD

The present invention is encompassed within the field of systems for taking measurements in a part of a digestive system.

### BACKGROUND

Inflammatory and immune-based diseases of the gastrointestinal tract, such as ulcerative colitis and Crohn's disease in humans, are increasingly present and occur at younger ages. These conditions are thought to result from abnormal activation of the immune system in a genetically vulnerable individual against components of the intestinal microbiota, which triggers inflammation leading to intestinal damage. Although the exact cause is not fully understood, it is generally accepted that a complex interplay between the host immune system, genetics, microbiota, and environmental factors is the primary causative agent. This includes an imbalance between proinflammatory and antiinflammatory cytokines and changes in the composition and function of the gut microbiota (dysbiosis), which are crucial. In summary, inflammatory gastrointestinal tract diseases (IGTDs) are caused by abnormal activation of the immune system in a genetically vulnerable individual against elements of the enteric microbiota, resulting in inflammation that causes intestinal damage. Therefore, early detection and treatment of these disorders depends on effective monitoring of relevant biomarkers in the gastrointestinal (GI) tract. Equivalently, in the treatment of diseases affecting specific animal species, the monitoring of biomarkers within their gastrointestinal tract is required.

It is already known in the art the use of ingestible electronics to track and treat gastrointestinal diseases. However, measurement techniques used in ingestible electronics can be further enhanced because known techniques have difficulties, for example, interferences with other analytes, electrical drift or low signal-to-noise ratio.

### SUMMARY

A first aspect of the disclosure relates to a system comprising:

- a swallowable or suckable device for taking measurements in a part of a digestive system, the device comprising:

  ○ a sensor configured to measure a biomarker by providing measurements of electrical parameters;
  ○ at least one of a data storage and a wireless communication module for receiving measurements taken by the sensor, the wireless communication module being configured to transmit data outside of the device;
  ○ an energy source for providing energy to the sensor and to the at least one of a data storage and a wireless communication module; and
  ○ a cover, the cover housing the sensor, the energy source and the at least one of a data storage and a wireless communication module;

- processing means configured to receive the measurements of the sensor and to process the measurements to estimate a presence of a biomarker.

The swallowable device is a pill for taking measurements, e.g., for taking measurements in a large intestine, after being swallowed by a user, e.g., a person or an animal (e.g., a cow, a pig or a dog).

The suckable device is a device for taking measurements in a mouth. It can be suck by a user, e.g., by a person or an animal (e.g., a cow, a pig or a dog), as if the device were candy.

Thereby, the part of the digestive system is, for example, a mouth or a large intestine.

Thereby, the sensor is configured to measure a biomarker present in the digestive system, for example, at least one of: fecal calprotectin, a cytokine indicative of antiinflammatory response (e.g., interleukin-6 or interleukin-12) and a cytokine indicative of proinflammatory response (e.g., interleukin-10 or Tumor necrosis factor, i.e., TNF$\alpha$).

If the device comprises the data storage, the measurements can be stored in the device; if the device comprises the communication module, the measurements can be transmitted outside of the device.

Data obtained by the device may be stored in the data storage. The data stored in the data storage may be transmitted out of the device (e.g., to the processing means or to other processing means) through a wireless communication module. Alternatively and/or additionally, the data may be transmitted out of the device by a wired communication module connected to the data storage (e.g., after the device has been ejected by the organism). A wireless communication module is advantageous in the sense that allows retrieving the data from the data storage without connecting any wire. In addition, the wireless communication module configured to transmit data outside of the device allows accessing the data obtained in the device relatively quickly after having obtained said data (i.e., while the device is in the digestive system) without requiring introducing inconvenient wires in the digestive system.

In some embodiments, the wireless communication module is configured to transmit data outside of a body of the user while the device is in the digestive system of the body. This may be achieved by a communication module configured to have a transmission power enough to reach a receiving device located outside of the body. An example of said communication may be Bluetooth.

Instead of using the data storage, the wireless communication module may be used because the wireless communication module allows transmitting the data out of the device without a previous storage in a data storage of the device.

The wireless communication module is particularly advantageous when the device is swallowable because allows obtaining data from the device while the device is in the digestive system. Thus, allows obtaining data relatively quickly after said data has been obtained by the device. In addition, the wireless communication module allows dispensing with the need to search and manipulate the device after the device has been ejected by the organism to retrieve the data.

The processing means may be part of the sensor or separate from the sensor. The processing means processes the measurements of electrical parameters to enable enhancing accuracy of biomarker measurements by performing operations with the measurements of electrical parameters.

In some embodiments, the processing means is at least one of: housed by the cover and outside of the cover. If the processing means is housed by the cover and is not outside of the cover, the processing of the electrical parameters takes place in the device. If the processing means is outside of the cover and is not housed by the cover, the processing of the electrical parameters takes place outside of the cover. If some components of the processing means are housed by the cover whereas other components of the processing means are outside of the cover, some of the processing takes place in the device and some processing takes place outside of the device.

In some embodiments, the measurements of the electrical parameters are measurements of electrical parameters of biorecognition transduction of a biochemical signal caused by the biomarker.

In some embodiments, the sensor comprises a biotransducer; wherein the biotransducer is a sensor transducer, the sensor transducer being configured to convert a biochemical signal caused by the biomarker to an electronic signal for measuring the biomarker.

In some embodiments, the device is configured to take at least three electrical measurements with the sensor comprising: a measurement at a single voltage in a first voltage range in which it is expected that a cyclic voltammogram, CV, has an extreme value of current, a measurement at a single voltage lower than the first voltage range, and a measurement at a single voltage higher than the first voltage range; and the processing means is configured to fit the portion of oxidative scan or the portion of reductive scan of a CV to the at least three electrical measurements.

Since each measurement is taken at a single voltage, each measurement is associated with the voltage at which the measurement is taken, so that a pair of values respectively indicative of electrical measurement (e.g., current) and voltage is obtained for each measurement.

The measurements taken are not those of a scan of a cyclic voltammetry, but are measurements at merely some sparse single voltages which are chosen and controlled.

For example, the device is configured to take less than 281 measurements, for example, less than 16 measurements for example, just three electrical measurements. The measurements may be taken in a voltage window of at least 0.3V.

The presence of the biomarker may be estimated by calculating an area under the oxidative part of the CV.

Thereby, the method can be understood as a combination of chronoamperometry with at least one of cyclic voltammetry, linear sweep voltammetry, differential pulse voltammetry and square pulse voltammetry. In particular, the method allows applying chronoamperometry to obtain each of the at least three values of voltage and allows obtaining the estimation of the oxidative or reductive portion of the CV with just as few as three measurements (i.e., without as many measurements as required in cyclic voltammetry/linear sweep voltammetry/differential pulse voltammetry/square pulse voltammetry).

Thereby, the method does not require that measurements are taken at a particular V/s together with taking measurements at a determined number of pairs current-voltage. Instead, the method merely requires taking measurements at determined single values of voltage, e.g., 1 V, 1.1V and 1.2 V, which do not require to be uniformly distributed in the scan. Preferably several measurements are taken in each one of the voltages, for example, measurements are taken during a determined time (e.g., five seconds) in 1 V, measurements are taken during a determined time (e.g., five seconds) in 1.1 V, and measurements are taken during a determined time (e.g., five seconds) in 1.2 V.

For conciseness, the voltage range in which it is expected that a cyclic voltammogram, CV, has an extreme value of current has been named the first voltage range.

By fitting the cyclic voltammogram to the at least three measurements, an estimation of the presence of the biomarker is enhanced by enhancing robustness of the estimation because, by fitting the curve, the estimation considers displacement of the extreme current of the CV, so that the estimation is more robust to said displacements. In addition, since the estimation of the CV merely requires three values, the CV may be estimated with just a few measurements of the sensor.

The skilled person in the field of biosensors is aware of several ways of determining the voltage range in which it is expected that the CV has an extreme value of current. For example, the redox potential in ideal thermodynamic conditions can be calculated, and then said calculation can be adjusted with experimental tests under real conditions of functioning of the sensor to enhance the estimation of the voltage range.

In some embodiments, the measurement at the voltage in the first voltage range is the voltage in which it is expected that the cyclic voltammogram, CV, has an extreme value of current.

Said voltage can be determined in a similar way as the voltage range in which it is expected that the CV has an extreme value of current.

In some embodiments, the at least three measurements are taken for the top part of the CV (i.e., for the portion of oxidative scan), and the extreme value is a maximum of current. In some embodiments, the at least three measurements are taken for the bottom part of the CV (i.e., for the portion of reductive scan), and the extreme value is a minimum of current.

By integrating an area delimited by the CV, for example, an area in the oxidative part of the CV, a value of power is obtained, the value of power being proportional to an amount of detected electroactive substance. Since the value of power is mathematically related to a concentration of the biomarker, the concentration of the biomarker can be calculated based on the value of power. For example, the processing means may be configured to calculate the concentration of the biomarker.

In some embodiments, the mathematical relation is:

$$y = a + bx$$

wherein:

$y$ is the value of power obtained by integrating the curve,
$a$ is a constant value,
$b$ is a constant value, and
$x$ is a value of concentration of the biomarker.

The constant values $a$ and $b$ are obtained in a calibration of the mathematical relation.

In some embodiments, the voltage lower than the first voltage range is a voltage at which it is expected that the CV has a capacitive portion in a portion of oxidative scan or the voltage higher than the first voltage range is a voltage at which it is expected that the CV has a capacitive portion in a portion of reductive scan.

A capacitive portion of the CV is a substantially straight portion of the CV which ends at a substantially exponential change of current. Taking a measurement(s) of the capacitive portion allows enhancing estimation of the capacitive portion.

The voltage at which it is expected that the CV has a capacitive portion in the portion of oxidative scan or in the portion of reductive scan can be determined in a similar way as the voltage range in which it is expected that the CV has an extreme value of current. In addition, the voltage at which it is expected that the CV has a capacitive portion can be estimated with complementary techniques, such as, electrochemical impedance.

In some embodiments, the voltage higher than the first voltage range is a voltage at which it is expected that the CV has plateau in a portion of oxidative scan or the voltage lower than the first voltage range is a voltage at which it is expected that the CV has a plateau in a portion of reductive scan.

A plateau is a region of the CV in which current does not change or has a relatively small change with change of voltage, the plateau is at the end of the portion of oxidative scan in the CV (i.e., highest voltages) or at the end of the reductive scan in the CV (i.e., lowest voltages). Taking measurement(s) in the plateau allows enhancing estimation of the portion of the CV different than the capacitive portion.

The voltage at which it is expected that the CV has plateau in the portion of oxidative scan or in the portion of reductive scan can be determined in a similar way as the voltage range in which it is expected that the CV has an extreme value of current.

In some embodiments, several measurements are taken at the single voltage lower than the first voltage range, several measurements are taken at the single voltage in the first voltage range, and several measurements are taken at the single voltage higher than the first voltage range; and the processing means is configured to compute an average of the several measurements at the voltage lower than the first voltage range, an average of the several measurements taken at the voltage in the first voltage range, and an average of the several measurements at the voltage higher than the first voltage range; and the processing means is configured to fit a CV to the at least three average values. By calculating average values, the accuracy of the estimation can be improved. This is achieved by reducing the impact of random noise and errors in the measurements through the combination of multiple measurements, thereby potentially yielding more accurate results. This technique, known as ensemble averaging, is a common practice in signal processing. It is based on the assumption that the measurements are independent and identically distributed, and that any errors present are random and not systematic in nature.

In some embodiments, the processing means is configured to perform the fitting to the CV by:

○ if the extreme value is a maximum of current:

■ estimating a capacitive portion of the CV based on:

○ the measurement or an average of measurements at the voltage lower than the first voltage range, and
○ a relation between a line and the measurement or the average of measurements at the voltage lower than the first voltage range;

■ applying an algorithm for determining a gaussian curve having a width based on the biomarker; and
■ determining a line joining the estimated capacitive portion with the gaussian curve;

○ if the extreme value is a minimum of current:

■ estimating a capacitive portion of the CV based on:

○ the measurement or an average of measurements at the voltage higher than the first voltage range, and
○ a relation between a line and the measurement or the average of measurements at the voltage higher than the first voltage range;

■ applying an algorithm for determining a gaussian curve having a width based on the biomarker; and
■ determining a line joining the estimated capacitive portion with the gaussian curve.

In some embodiments, the relation between a line and the measurement or the average of measurements at the voltage lower than the first voltage range, is a relation between said measurement or average of measurements and one of: a straight line, a polynomial line or an exponential line. The type of line may depend on factors, such as, the analyte/biomarker, electrode configuration, composition of the matrix and redox behavior of a species.

In some embodiments, the line joining the estimated capacitive portion with the gaussian curve is determined based on the estimated capacitive portion and the gaussian curve.

In some embodiments, the gaussian curve joins the extreme value with the plateau. The point which includes the measurement at a voltage in the plateau may be used for checking that the width of the gaussian curve is appropriate. In some embodiments, the point which includes the measurement at a voltage in the plateau is not used for determining the line joining the gaussian curve with the plateau.

In some embodiments, the device comprises an additional sensor; the processing means is configured to receive measurements taken by the additional sensor; and the processing means is configured to identify determined measurements of the additional sensor as indicative of anomaly.

Thereby, if, based on the measurements taken by the additional sensor, there is an anomaly in the functioning of the device, the processing means determine that there is an anomaly. For example, the anomaly may be at least one of: wrong functioning of electronics involved in taking measurements, undue reduction of passage of fluid inside the cover and to the sensors, too much electrical noise, unexpected molecules reaching the sensor(s), expected molecules not reaching the sensor(s) and drift of measurements of the sensor(s).

In some embodiments, the processing means is configured to, upon identifying that a received measurement is indicative of an anomaly, not estimating the presence of the biomarker. Thereby, since conditions required to perform the estimation may not be fulfilled, the estimation is not performed.

In some embodiments, the processing means is configured to, upon identifying that a received measurement is indicative of an anomaly, sending a warning signal to an actuator configured to emit a signal perceivable by a human based on the warning signal. The actuator is, for example, a display or a loudspeaker which, respectively, displays something or emits sound based in the signal.

In some embodiments, the additional sensor comprises a bioreceptor to detect a first analyte different than the biomarker, so that the additional sensor is configured to provide measurements indicative of a presence of the first analyte; the first analyte being in the part of the digestive system.

A bioreceptor is a biological element (e.g., enzyme or antibody) which is sensitive to recognizing the analyte (e.g.,

enzyme substrate or complementary DNA).

In some embodiments, the bioreceptor is at least one of an aptamer, a nanobody and an antibody.

The device is for taking measurements in a particular part of the digestive system, the additional sensor is configured to measure an analyte present at least in said part. For example, if the device is a pill for taking measurements in the large intestine, the analyte may be Immunoglobulin G. Thereby, if the measurement of the additional sensor is indicative of absence of Immunoglobulin G, when it is expected that there is Immunoglobulin G, it is indicative of an anomaly.

The fact that the bioreceptor is to detect a first analyte different than the biomarker does not exclude that the bioreceptor may also to detect the biomarker and/or that the biomarker may interfere in the measurements of the first analyte. However, if the detection of the analyte (e.g., immunoglobulin G) takes place by means of a bioreceptor which is blocked (absence of non-specific detection), the bioreceptor should only detect immunoglobulin G (e.g., the bioreceptor should not detect calprotectin).

In some embodiments, the additional sensor comprises an agent to block interaction of the additional sensor with the biomarker and to detect at least a second analyte different than the biomarker, so that the additional sensor is configured to provide measurements indicative of a presence of the at least second analyte; the at least a second analyte being in the part of the digestive system.

In some embodiments, the blocking agent (i.e., the agent to block) is at least one of serum albumin and fluorosilane. The blocking agent ensures that the additional sensor does not measure the biomarker because the blocking agent blocks interaction of the additional sensor with the biomarker. An additional sensor with a blocking agent allows measuring the at least a second analyte thus allowing obtaining a background signal. The background signal allows detecting capacitive or non-Faradaic signals caused by biomolecules reaching the additional sensor. These signals increase or decrease current detected below the peak of the CV so that the signal may mask or distort the peak of the CV. The background signal also allows detecting that electroactive substances (i.e., species exhibiting electronic transitions) reach the additional sensor since it is known that the signals derived from the presence of said substances do not belong to the redox standard because the signal does not appear at the characteristic voltage of the redox mediator used. Combining the signal of the additional sensor with the signal of the sensor allows minimizing, and even preventing, masking which can cause an error in the estimation of the biomarker.

In some embodiments, the cover comprises a portion made of a material configured to be disintegrated at a determined pH range, wherein a detecting part of the sensor is covered by the portion of the cover so that the detecting part is exposed when the portion of the cover is disintegrated.

The material of the portion of the cover allows that fluid does not reach the detecting part of the sensor until the cover reaches a place in which the pH is such that the portion of the cover is disintegrated. Thus, the biomarker can only reach the detecting part of the sensor after the portion of the cover is disintegrated, thus the sensor can obtain measurements of the biomarker merely after the portion of the cover is disintegrated. Thereby, a portion of the cover of said material allows certain control of the places of the body in which a corporal fluid reaches the detecting part of the sensor.

In some embodiments, the portion of the cover is configured to be disintegrated at a pH of at least 5. Normally, a pH of at least 5 is not reached until the cover arrives at the small intestine. Therefore, it allows taking measurements from the small intestine and not before reaching the small intestine.

In some embodiments, the portion of the cover is configured to be disintegrated on a pH of at least 7, preferably of at least 8. Normally, a pH of at least 7 is not reached until the cover arrives at the large intestine or the end portion of the small intestine, and a pH of at least 8 is not reached until the large intestine. Therefore, it allows taking measurements from the large intestine and/or the end of the small intestine and not before reaching said parts of the digestive system.

Some examples of materials of the portion are those used in enteric coatings, e.g., fatty acids, waxes, plant fibers, natural polysaccharides, animal proteins and synthetic acrylic copolymers.

In some embodiments, a detecting part of the additional sensor is covered by the portion so that the detecting part of the additional sensor is exposed when the material of the portion of the cover is disintegrated.

In some embodiments, the cover comprises a portion made of a material configured to be disintegrated with saliva, and a detecting part of the sensor is covered by the portion of the cover so that the detecting part is exposed when the portion of the cover is disintegrated.

The material of the portion of the cover allows that fluid does not reach the detecting part of the sensor until the cover is in the mouth for a while so that the portion of the cover is disintegrated. Thus, the sensor can obtain measurements of the biomarker merely after the portion of the cover is disintegrated.

Some examples of the materials are hydrophilic animal proteins, natural polysaccharides and synthetic hydrophilic polymers (i.e., gelatin, alginate, sugars, polyvinylalcohol and polyvinylpyrrolidone).

In some embodiments, a detecting part of the additional sensor is covered by the portion of the cover so that the detecting part of the additional sensor is exposed when the material of the portion of the cover is disintegrated.

In some embodiments, the device comprises a filter between a detecting part of the sensor and the portion of the cover, and the filter is configured to allow the pass of a fluid containing the biomarker.

Thereby, fluid reaches the filter merely after the portion of the cover has been disintegrated.

The filter is configured to prevent and/or minimize chances that determined analytes reach the detecting part of the sensor. Certain analytes may undesirably change measurements of the sensor, so that wrong concentrations of the biomarker may be obtained based on measurements of the sensor.

In some embodiments, the filter is a membrane.

In some embodiments, the filter is made of a hydrophilic material. Hydrophilia allows enhancing the passing of fluid through the filter. Preferably, the hydrophilic material is also for preventing adhesion of proteins. For example, the filter may be made of polyethylene glycol.

In some embodiments, the filter comprises an antifouling coating.

The antifouling coating allows preventing and/or minimizing chances that the filter clogs, for example, prevents aggregation of microbes to prevent formation of a biofilm which clogs the filter.

In some embodiments, the coating comprises antimicrobial enzymes (i.e., DNase I) or biocidal cationic polymers (i.e., chitosan and poliethylenimine).

A second aspect of the disclosure relates to a computer program comprising instructions which, when executed by the processing means of the system of the first aspect of the disclosure, cause the processing means to receive the measurements of the sensor of the system and to process the measurements to estimate a presence of a biomarker.

A third aspect of the disclosure relates to a computer-readable medium having stored thereon the computer program of the second aspect of the disclosure.

Similar advantages as those described with respect to the first aspect of the disclosure are also applicable to the second aspect of the disclosure and to the third aspect of the disclosure.

## BRIEF DESCRIPTION OF THE DRAWINGS

To complete the description and to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:

Figure 1 shows a system in accordance with embodiments.

Figure 2A shows a swallowable or suckable device in accordance with embodiments. Figure 2B shows the same swallowable or suckable device as figure 2A but without the antenna to better show an energy source of the device.

Figure 3 shows a swallowable or suckable device in accordance with embodiments.

Figure 4 shows a cyclic voltammogram, CV.

Figure 4A shows the portion of oxidative scan of the CV of figure 4 and background signals.

Figure 4B shows the portion of reductive scan of the CV of figure 4.

Figure 5 shows a background signal of a voltametric peak; wherein the initial portion of the background signal is indeed the capacitive portion of an oxidative scan of a CV.

Figure 6 shows an area which can be calculated to estimate a presence of a biomarker.

## DETAILED DESCRIPTION

The following description is not to be taken in a limiting sense but is given solely for the purpose of describing the broad principles of the invention. Embodiments of the invention will be described by way of example, with reference to the above-mentioned drawings.

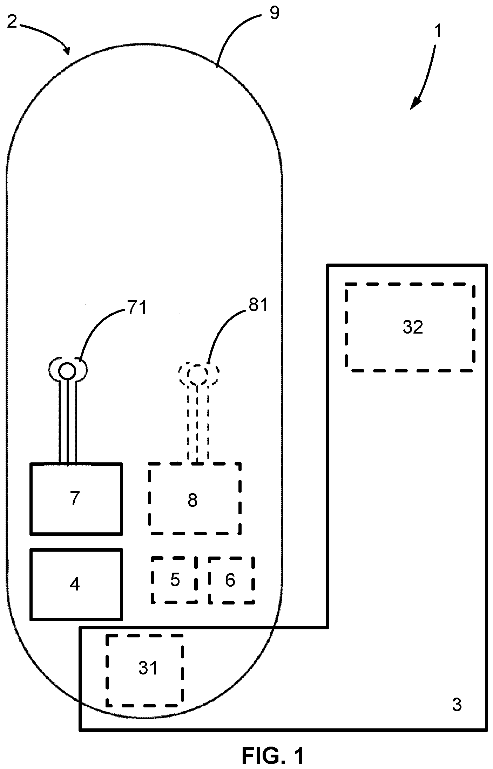

Figure 1 shows a system 1 in accordance with embodiments. The system 1 comprises a swallowable or suckable device 2. The device 2 comprises a cover 9. The device 2 can be swallowable or suckable depending on the material of a portion of the cover 9. If the material disintegrates with saliva the device is suckable. If the material of the portion of the cover does not disintegrate with saliva but is configured to be disintegrated in a part of the digestive system different than the mouth (e.g., due to a particular pH of said part of the digestive system), then the device 2 is swallowable.

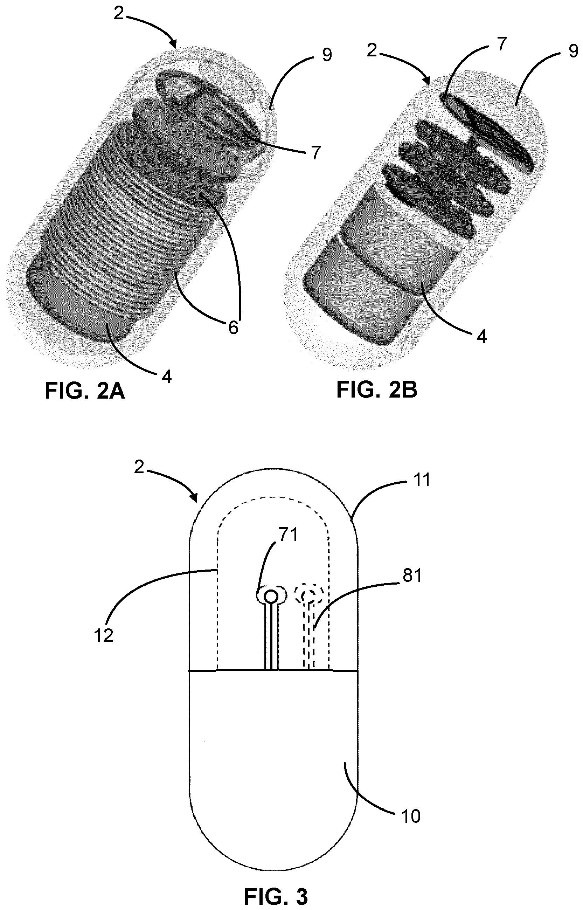

The device 2 comprises a sensor 7 for measuring a biomarker. The sensor 7 comprises a detecting part 71. The sensor 7 is configured to measure the biomarker when a digestive system fluid which contains the biomarker reaches the detecting part 71. The detecting part 71 comprises a bioreceptor, for example, an aptamer or a nanobody. Figure 1 shows a sensor provided with a cell of three electrodes. However, other types of sensors could be used, for example, those provided with an organic electrochemical transistor (OEC), e.g., as illustrated in figure 2A.

The device 2 comprises at least one of a data storage 5 and a wireless communication module 6. The system 1 comprises processing means 3. The processing means 3 may comprise processing means 31 in the device 2 and/or processing means 32 outside of the device 2. Although the processing means 31 have been shown as components separated from sensor(s) 7, 8, the data storage 5 and the wireless communication module 6, in some embodiments, the processing means 31 can be considered to be part of the sensor(s) 7, 8, the data storage 5 and/or the wireless communication module 6.

If the device 2 comprises the data storage 5, the device 2 is configured to store measurements of electrical parameters provided by the sensor 7 and/or data resulting from processing the measurements, the processing being performed in the device 2 by the processing means 31. To receive said data, the data storage 5 may be communicatively coupled with the sensor 7 and/or with the processing means 31.

If the device 2 comprises the wireless communication module 6, the device 2 is configured to transmit the electrical measurements provided by the sensor 7 and/or data resulting from processing the measurements, the processing being performed in the device 2 and by the processing means 31. To receive said data, the wireless communication module 6 may be communicatively coupled with the sensor 7 and/or with the processing means 31.

If the device 2 is a suckable device, the device 2 may comprise both the data storage 5 and the wireless communication module 6. The wireless communication module 6 may be for Near-field communication, so that after the device 2 has taken measurements in the mouth, the device 2 is taken out of the mouth and brought near a receiving device to which the wireless communication module 6 sends the data previously obtained in the device 2 and stored in the data storage 5.

To process the data with the processing means 32 located outside of the body, the processing means 32 receives said data prior to the processing. The processing means 32 is communicatively coupled with the wireless communication module 6 to receive said data from the wireless communication means and/or the processing means 32 is communicatively couplable with the data storage 5 to receive said data from the data storage 5.

The device 2 optionally comprises an additional sensor 8. The additional sensor 8 may be used as at least one of: control sensor to determine if there is any anomaly which cause that measurements of the sensor 7 are wrong, and as a correction sensor to correct measurements of the sensor 7.

The additional sensor 8 is communicatively coupled with at least one of the data storage 5, the wireless communication module 6 and the processing means 31, so that measurements of the additional sensor 8 may be processed by the processing means 3.

The device 2 comprises an energy source 4 for providing energy to the sensor 7, to the additional sensor 8 if the device 2 comprises the additional sensor 8, to the at least one of a data storage 5 and a wireless communication module 6, and to the processing means 31 if the device 2 comprises the processing means 31.

From the above description, it is apparent that, in those embodiments in which the processing means 3 comprises the processing means 31 and the processing means 32, the at least one of data storage 5 and a wireless communication module 6 may allow transmitting data from the processing means 31 to the processing means 32 and/or transmitting data from the processing means 32 to the processing means 31.

Figure 2A shows components of the device 2 within the cover 9. In particular, figure 2A shows the energy source 4, the wireless communication module 6, the sensor 7 and the cover 9. Figure 2B shows a view of the device 2 of figure 2A in which an antenna of the communication module 6 has been removed for better showing the energy source 4.

Figure 3 shows details of a cover of the device 2 as well as of a location of a detecting part 71 of the sensor 7 and of a detecting part 81 of the additional sensor 8.

The cover 9 may comprise two portions 10, 11 made of different materials. A first portion 11 of the cover 9 is made of material which disintegrates in contact with a fluid of a digestive system (e.g., which disintegrates with saliva and other fluids with extreme pH within the digestive system). A second portion 10 of the cover 9 is made of a material which does not disintegrate in contact with a fluid of the digestive system (e.g., resistant to both saliva and other fluids with extreme pH along the digestive system).

The second portion 10 covers components of the device 2 for blocking passage of fluid to the components covered by the second portion 10 (e.g., to the energy source, to the processing means 31, to the at least one of a data storage 5 and a wireless communication module 6). Between the second portion 10 and the detecting part(s) 71, 81, there may be a covering for blocking passage of fluid after the first portion 11 has been disintegrated.

The first portion 11 covers the detecting parts 71, 81 of the sensors 7, 8. Thereby, when the first portion 11 is disintegrated, fluid can reach the detecting parts 71, 81.

The device 2 may comprise a filter 12 between the first portion 11 of the cover and the sensors 7, 8, in particular, the detecting parts 71, 81 of the sensors). The filter 12 performs the filtering according to size of particles to be filtered. The filter 12 allows passage of the biomarkers. The filter 12 also allows passage of analytes which do not interfere in the bioanalytic detection or which interference can be corrected with the measurements of the additional sensor 8. Thereby, the filter 12 allows removing measurement interferences caused by an uncontrolled presence of analytes which reach the detecting parts 71, 81.

The filter 12 may be made of a hydrophilic material which prevents adhesion of proteins, e.g., polyethylene glycol.

The filter 12 may comprise an antimicrobial coating for preventing formation of a biofilm on the filter 12.

After introducing the device in the mouth, if the device 2 is a suckable device, a user sucks the device to remove the first portion 11 of the cover 9, so that fluid of the mouth reaches the detecting part 71, 81 of the sensor(s) 7, 8.

If the device 2 is a swallowable device, a user swallows the device after introducing it in the mouth. By making the first portion 11 of the cover 9 of a suitable material, it can be controlled in which part of the digestive system the first portion 11 is disintegrated, so that fluid can reach the detecting part(s) 71, 81 after the first portion is disintegrated.

The detecting part 71 of the sensor 7 may comprise an organic electrochemical transistor or a cell of two or more electrodes. A bioreceptor of the detecting part 71 may be chemically coupled to the cell of the two or more electrodes, or may be chemically coupled to the PEDOT:PSS channel of the organic electrochemical transistor.

The sensor 7 may comprise an electrochemical transducer for converting a biochemical signal caused by interaction of the biomarker with the bioreceptor to an electronic signal (e.g., a signal indicative of voltage and/or current). Although in the description below the only measurements of electrical parameters are measurements of current considering cyclic voltammetry technique, the skilled person understands that other electrical parameters could be taken with the sensor to estimate a presence of a biomarker. For example, impedance-based measurements over a range of frequencies could be obtained using an electrochemical transduction technique since the binding of a biomarker to the bioreceptor causes a change in the electrical impedance of the bioreceptor-biomarker complex, resulting in a detectable impedance shift. When the biomarker (i.e., a fluid comprising the biomarker) reaches the detecting part 71 of the sensor 7, a biochemical signal caused by interaction of the biomarker with the bioreceptor is converted to an electronic signal indicative of a measurement of an electrical parameter.

The measurements of electrical parameters may be measurements of current obtained by voltammetry. Each measurement of current is obtained for a determined voltage. Thereby, for each current measurement, a pair voltage and current measured at said voltage is obtained.

The processing means 3 receive the electrical measurements (e.g., the pairs of voltage-current) and processes the electrical measurements to estimate a presence of the biomarker, for example, to estimate a concentration of a biomarker in a fluid reaching the detecting part 71. The estimation of the presence of the biomarker is based on a cyclic voltammogram, CV, calculated by the processing means based on the obtained measurements, for example, based on the pairs of current-voltage.

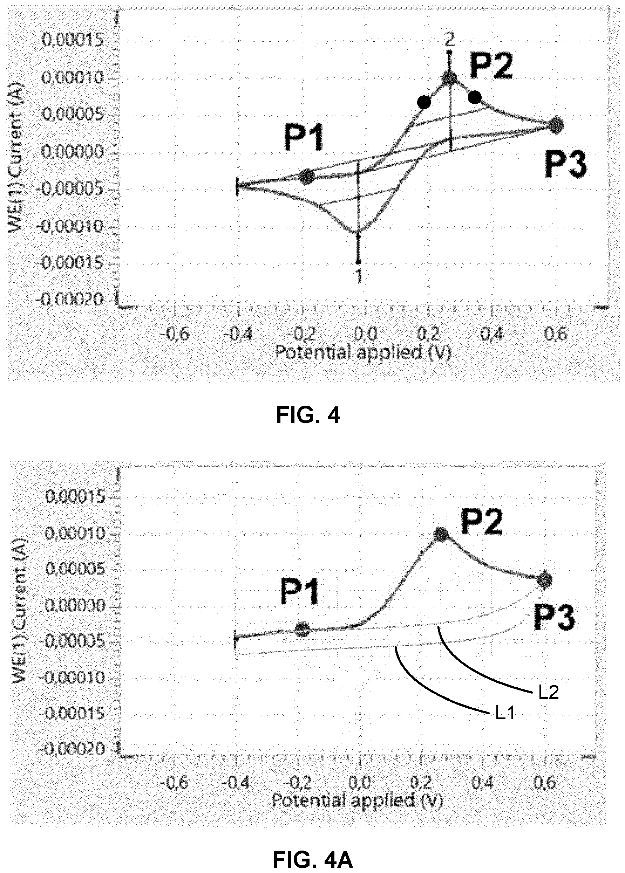

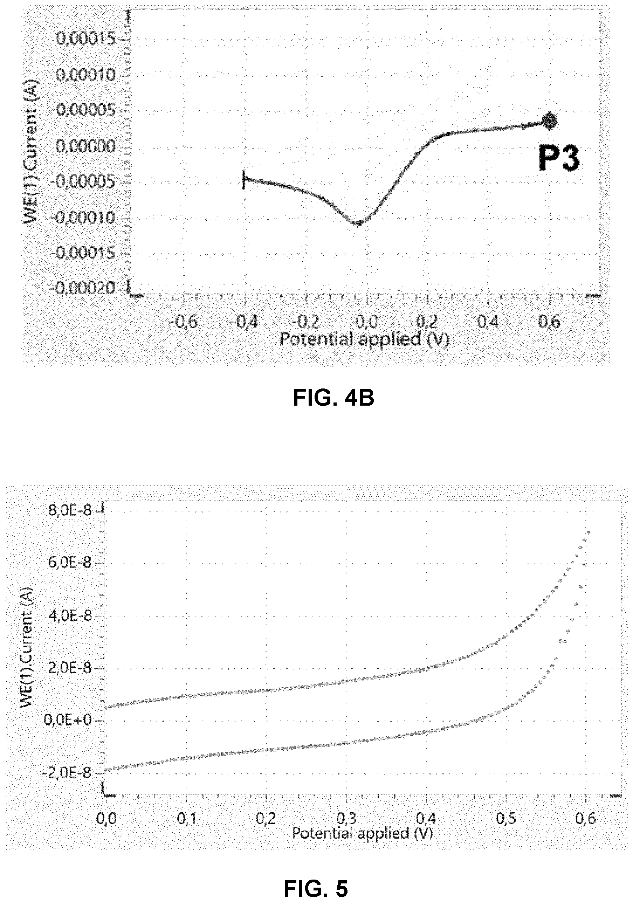

To estimate the presence of the biomarker, measurements at at least three different single voltages are taken: a measurement at a single voltage in a first voltage range in which it is expected that the CV has an extreme (i.e., maximum or minimum) value of current, a measurement at a voltage lower than the first voltage range, and a measurement at a voltage higher than the first voltage range. Examples of the three different single voltages are illustrated in figure 4, which shows a first pair P1 of voltage-current, a second pair P2 of voltage-current and a third pair P3 of voltage-current. In the example of figure 4, the measurements have been taken in the oxidative scan portion of the CV, thus the extreme value of current is a local maximum of current. If the measurements were taken in the reductive scan portion of the CV, the extreme value of current would be a local minimum of current.

To enhance estimation of the CV, each pair of values may comprise an average value of current measured at the voltage of the respective pair. For example, for each one of the at least three single voltages several measurements of current may be taken along a determined period of time or a determined number of measurements may be taken, and said measurements may be averaged to define the respective pair of voltage and average current.

The CV shown in figure 4 is a CV used for estimation of presence of fecal calprotectin, wherein the CV has been estimated from just three pairs P1, P2, P3 of voltage-current values. The CV of figure 4 corresponds to a detecting part 71 comprising an aptamer marked with a redox standard of ferrocene. Oxidation of ferrocene happens at a relatively low voltage with respect to the reference electrode, e.g., at about 0.5 V with respect to a saturated calomel electrode of reference. In Ferrocene a quick electronic transition causes a stable cation:

$$Fe(C_2H_5)_2 \Leftrightarrow Fe(C_2H_5)^+_2 + e\text{-}$$

The voltage at which the peak of electrochemical detection happens can be derived from the Nernst equation. In this equation the voltage depends on the concentration of biomarker that reaches the detecting part 71 of the sensor 7. Thereby, when the concentration changes, the extreme value of current moves to higher or lower voltages depending on the standard voltage (i.e., voltage in ideal conditions). In the case of detection of calprotectin, the voltage at which the current peak happens depends on the concentration of calprotectin in the respective part of the digestive system.

The CV may be fitted to P1, P2 and P3 in the following manner:

P1 is deemed to belong to the capacitive portion of the CV. A line relating current to voltage (e.g., a determined function relating current to voltage) may be adjusted based on P1. The line may be defined between voltages of values -0.4 V and 0 V. Thus, the capacitive portion may be estimated adjusting the line using P1 so that P1 belongs to the estimation of the capacitive portion. The line may have been obtained based on a coefficient of determination and previously to the adjustment.

The portion of the CV near the maximum value of current may be estimated by fitting a gaussian curve to a gaussian width obtained from the type of biomarker. The gaussian width may be obtained from the equation:

$$\Delta E = 0.059 \; x \; n$$

wherein:

ΔE is a width of the gaussian curve; and
n is the number of electrons transferred in the redox reaction.
ΔE may be obtained from the Nernst equation which governs thermodynamics of the redox reaction, in which the value of the peak is in equilibrium. The Nernst equation is:

$$\Delta E \; = \; 2.218 \; x \; n \; RT/F$$

wherein:

n is the number of electrons;
F is the Faraday constant;
T is the temperature; and
R is the gas constant.

$$\Delta E = 59 \text{ mV at ambient temperature and ideal conditions}$$

The above parameter ΔE is theoretical and in the reality can be slightly different.

The shape of the CV may be governed by a combination of the Nernst equation and the Fick's law, assuming a process controlled by diffusion. The combination of both is expressed as a function of current and time by means of a semi-integration process which provides the Randless-Sevick equation, which is the equation used for defining the gaussian curve:

$$I \, p = \; 0.4463 \; [(n^{\wedge}(3)F^{\wedge}(3)vDp)/(RT)]^{\wedge}(1/2) \; A$$

wherein:

n is the number of electrons;
F is the Faraday constant;
v is the scan speed;
T is the temperature;
R is the gas constant; and
A is the electroactive area of the electrode.

The gaussian curve can be determined processing the equation of the gaussian distribution:

$$I(E) = Ip \; {}^{*} exp(-((E - Ep) / (\Delta E / 2.355))^{\wedge}2)$$

wherein:

$$\Delta E = 0.059 \; x \; n$$

The of the CV between the capacitive portion and the gaussian curve can be estimated by the intersection of a continuation of the line of the capacitive portion and a continuation of the gaussian curve. The continuation is defined by the mathematical function defining each one of the line of the capacitive portion and the gaussian curve.

P3 can be used to check that the estimation of the peak of the CV is correct. In particular, P3 is a point which can be used to check that the width of the gaussian curve corresponds to the theoretical 0.059n value used in the algorithm.

The fitting to the CV may be obtained using machine learning and/or neural networks. For example, using the algorithms disclosed in Du, L., Thoma, Y., Rodino, F., & Carrara, S. (2024). Automatic simulation of electrochemical sensors by machine learning for drugs quantification. Electrochimica Acta, 491, 144304, or the algorithms disclosed in Gu, B. C., Chung, K. J., Chen, B. W., Dai, Y. H., & Wu, C. C. (2023). Electrochemical detection combined with artificial neural networks for the simultaneous intelligent sensing of caffeine and chlorogenic acid. Electrochimica Acta, 463, 142820.

The additional sensor 8 can be one of the following sensors or a combination of both. A first possibility is that the additional sensor 8 comprises a detecting part 81 comprising a bioreceptor. The bioreceptor is configured to detect a first analyte different than the biomarker, so that the additional sensor is configured to provide measurements indicative of presence of the first analyte. Since the first analyte is present in the same part of the digestive system as the portion in which the sensor 7 takes measurements of the biomarker, an anomaly can be detected with the additional sensor 8 when the additional sensor 8 does not provide measurements indicative of presence of the first analyte in said part of the digestive system.

A second possibility is that the additional sensor 8 comprises a detecting part 81 comprising an agent to block interaction of the additional sensor 8 with the biomarker and to detect at least a second analyte different than the biomarker, so that the additional sensor is configured to provide measurements indicative of presence of the at least second analyte, wherein the at least second analyte is in the part of the digestive system in which the sensor 7 will take measurements of the biomarker. Since this additional sensor provides measurements of at least a second analyte different than the biomarker, the additional sensor 8 provides measurements of electrical parameters which are caused by said at least second analyte. Therefore, if the electrical measurements of the additional sensor 8 are subtracted to the electrical measurements of the sensor 7, the influence of said at least second analyte in the measurements of the sensor is minimized or removed. Thereby, the additional sensor 8 allows minimizing noise of the electrical measurements of the sensor 7.

Figure 5 shows electrical measurements obtained with an additional sensor 8 of the second possibility, thus depicting a background signal. The current values of the background signal are due to capacitive features of the system (e.g., measured solution and content of ions). The values of current of the background signal shown in figure 5 are much lower than the values of current in the CV of figure 4, since they correspond to a case different from the one of figure 4. In fact, figure 4A already shows the lines L1, L2 of measurements of an additional sensor 8 for the case of figure 4A.

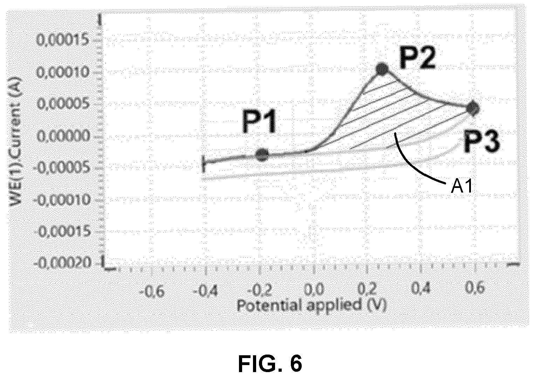

Figure 6 shows an example of an area A1 defined in the oxidative scan of the CV which may be calculated to estimate the presence of a biomarker. The presence of the biomarker can be estimated in an analogous manner calculating an area defined in the reductive scan of the CV.

The processing means may comprise, for example, at least one CPU, at least one GPU, at least one FPGA, at least one ASIC, at least one personal computer, at least one laptop, etc.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

**Claims**

1. A system (1) comprising:

   • a swallowable or suckable device (2) for taking measurements in a part of a digestive system, the device (2) comprising:

      ◦ a sensor (7) configured to measure a biomarker by providing measurements of electrical parameters;
      ◦ at least one of a data storage (5) and a wireless communication module (6) for receiving measurements taken by the sensor (7), the wireless communication module (6) being configured to transmit data outside of the device (2);
      ◦ an energy source (4) for providing energy to the sensor (7) and to the at least one of a data storage (5) and a wireless communication module (6); and
      ◦ a cover (9), the cover (9) housing the sensor (7), the energy source (4) and the at least one of a data storage (5) and a wireless communication module (6);

   • processing means (3) configured to receive the measurements of the sensor (7) and to process the measurements to estimate a presence of a biomarker.

2. The method of claim 1, wherein the measurements of the electrical parameters are measurements of electrical parameters of biorecognition transduction of a biochemical signal caused by the biomarker.

3. The system of any one of the previous claims , wherein the device (2) is configured to take at least three single electrical measurements with the sensor comprising: a measurement at a single voltage in a first voltage range in which it is expected that a cyclic voltammogram, CV, has an extreme value of current, a measurement at a single voltage lower than the first voltage range, and a measurement at a single voltage higher than the first voltage range; and the processing means is configured to fit the portion of oxidative scan or the portion of reductive scan of a CV to the at least three electrical measurements.

4. The system of claim 3, wherein the voltage lower than the first voltage range is a voltage at which it is expected that the CV has a capacitive portion in a portion of oxidative scan or the voltage higher than the first voltage range is a voltage at which it is expected that the CV has a capacitive portion in a portion of reductive scan.

5. The system of claim 3 or 4, wherein the voltage higher than the first voltage range is a voltage at which it is expected that the CV has plateau in a portion of oxidative scan or the voltage lower than the first voltage range is a voltage at which it is expected that the CV has a plateau in a portion of reductive scan.

6. The system of any one of claims 3 to 5, the processing means being configured to perform the fitting to the CV by:

   ○ if the extreme value is a maximum of current:

      ▪ estimating a capacitive portion of the CV based on:

         ○ the measurement or an average of measurements at the voltage lower than the first voltage range, and
         ○ a relation between a line and the measurement or the average of measurements at the voltage lower than the first voltage range;

      ▪ applying an algorithm for determining a gaussian curve having a width based on the biomarker; and
      ▪ determining a line joining the estimated capacitive portion with the gaussian curve;

   ○ if the extreme value is a minimum of current:

      ▪ estimating a capacitive portion of the CV based on:

         ○ the measurement or an average of measurements at the voltage higher than the first voltage range, and
         ○ a relation between a line and the measurement or the average of measurements at the voltage higher than the first voltage range;

      ▪ applying an algorithm for determining a gaussian curve having a width based on the biomarker; and
      ▪ determining a line joining the estimated capacitive portion with the gaussian curve.

7. The system of any one of the previous claims, wherein the device (2) comprises an additional sensor (8); the processing means (3) being configured to receive measurements taken by the additional sensor (8); the processing means (3) being configured to identify determined measurements of the additional sensor (8) as indicative of anomaly.

8. The system of claim 7, wherein the additional sensor (8) comprises a bioreceptor to detect a first analyte different than the biomarker, so that the additional sensor (8) is configured to provide measurements indicative of a presence of the first analyte; the first analyte being in the part of the digestive system.

9. The system of claim 7, wherein the additional sensor (8) comprises an agent to block interaction of the additional sensor (8) with the biomarker and to detect at least a second analyte different than the biomarker, so that the additional sensor (8) is configured to provide measurements indicative of a presence of the at least second analyte; the at least a second analyte being in the part of the digestive system.

10. The system of any one of the previous claims, wherein the cover (9) comprises a portion (11) made of a material configured to be disintegrated at a determined pH range, wherein a detecting part of the sensor (7) is covered by the portion (11) of the cover so that the detecting part is exposed when the portion (11) of the cover is disintegrated.

11. The system of any one of claims 1 to 9, wherein the cover (9) comprises a portion (11) made of a material configured to be disintegrated with saliva, wherein a detecting part (71) of the sensor (7) is covered by the portion (11) of the cover so that the detecting part (71) is exposed when the portion (11) of the cover is disintegrated.

12. The system of claim 11, the device (2) comprising a filter (12) between a detecting part (71) of the sensor (7) and the portion of the cover (9), the filter (12) being configured to allow pass of a fluid containing the biomarker.

13. The system of claim 12, wherein the filter (12) comprises an antifouling coating.

14. A computer program comprising instructions which, when executed by the processing means (3) of the system (1) of any one of the previous claims, cause the processing means (3) to receive the measurements of the sensor (7) of the system and to process the measurements to estimate a presence of a biomarker.

15. A computer-readable medium having stored thereon the computer program of claim 14.

**FIG. 1**

FIG. 2A

FIG. 2B

FIG. 3

FIG. 4

FIG. 4A

FIG. 4B

FIG. 5

FIG. 6

Europäisches
Patentamt

European
Patent Office

Office européen
des brevets

# EUROPEAN SEARCH REPORT

**Application Number**

**EP 24 38 2899**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | STRAKER MICHAEL A ET AL: "Seropill: Novel Minimally Invasive Ingestible Capsule for Serotonin Sensing in the GI Tract", 2023 22ND INTERNATIONAL CONFERENCE ON SOLID-STATE SENSORS, ACTUATORS AND MICROSYSTEMS (TRANSDUCERS), IEEJ, 25 June 2023 (2023-06-25), pages 1884-1887, XP034600286, [retrieved on 2024-05-07] | 1-5, 12-15 | INV.<br>A61B5/07<br>A61B5/145 |
| A | * the whole document * | 6 | |
| X | JP 2009 240474 A (UNIV HIROSHIMA) 22 October 2009 (2009-10-22) | 1,2,10, 12,14,15 | |
| A | * figures 1-3 * | 6 | |
| X | US 2011/046458 A1 (PINEDO HERBERT MICHAEL [NL] ET AL) 24 February 2011 (2011-02-24) | 1,7,8, 14,15 | |
| A | * paragraphs [0036], [0037], [0059]; figures 1-3 * | 6 | |
| X | US 2008/208077 A1 (IDDAN GAVRIEL J [IL] ET AL) 28 August 2008 (2008-08-28) | 1,7-11, 14,15 | TECHNICAL FIELDS SEARCHED (IPC) |
| A | * paragraphs [0068] - [0075], [0122]; figure 14 * | 6 | A61B |

The present search report has been drawn up for all claims

| Place of search | Date of completion of the search | Examiner |
|---|---|---|
| The Hague | 21 January 2025 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 2899

This annex lists the patent family members relating to the patent documents cited in  the above-mentioned European search report.
The members are as contained in the European Patent Office EDP file on
The European Patent Office is in no way liable for these particulars which are merely given for the purpose of information.

21-01-2025

| Patent document cited in search report | Publication date | Patent family member(s) | | Publication date |
|---|---|---|---|---|
| JP 2009240474 A | 22-10-2009 | NONE | | |
| US 2011046458 A1 | 24-02-2011 | CN | 102006823 A | 06-04-2011 |
| | | EP | 2259714 A1 | 15-12-2010 |
| | | US | 2011046458 A1 | 24-02-2011 |
| | | WO | 2009104967 A1 | 27-08-2009 |
| US 2008208077 A1 | 28-08-2008 | AT | E481032 T1 | 15-10-2010 |
| | | AU | 2005245224 A1 | 01-12-2005 |
| | | EP | 1755982 A2 | 28-02-2007 |
| | | JP | 4709836 B2 | 29-06-2011 |
| | | JP | 2008500126 A | 10-01-2008 |
| | | US | 2008208077 A1 | 28-08-2008 |
| | | WO | 2005113374 A2 | 01-12-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DU, L.** ; **THOMA, Y.** ; **RODINO, F.** ; **CARRARA, S.** Automatic simulation of electrochemical sensors by machine learning for drugs quantification. *Electrochimica Acta*, 2024, vol. 491, 144304

- **GU, B. C.** ; **CHUNG, K. J.** ; **CHEN, B. W.** ; **DAI, Y. H.** ; **WU, C. C.** Electrochemical detection combined with artificial neural networks for the simultaneous intelligent sensing of caffeine and chlorogenic acid. *Electrochimica Acta*, 2023, vol. 463, 142820